(19) Europäisches Patentamt
European Patent Office
Office européen des brevets

(11) Veröffentlichungsnummer: **0 490 379 A2**

(12) # EUROPÄISCHE PATENTANMELDUNG

(21) Anmeldenummer: **91121351.0**

(22) Anmeldetag: **12.12.91**

(51) Int. Cl.5: **C07D 295/185**, A61K 31/395, A61K 31/195, A61K 37/02, C07K 5/06

(30) Priorität: **13.12.90 DE 4040184**
**07.06.91 DE 4119235**

(43) Veröffentlichungstag der Anmeldung:
**17.06.92 Patentblatt 92/25**

(84) Benannte Vertragsstaaten:
**AT BE CH DE DK ES FR GB GR IT LI NL SE**

(71) Anmelder: **BERLIN-CHEMIE Aktiengesellschaft**
**Glienicker Weg 125**
**O-1199 Berlin(DE)**

(72) Erfinder: **Niedrich, Hartmut, Prof. Dr.**
**Theodor-Brugsch-Strasse 38**
**O-1115 Berlin(DE)**
Erfinder: **Roske, Irmard, Dr.**
**Basdorfer Strasse 23**
**O-1140 Berlin(DE)**
Erfinder: **Nieber, Karen, Dr.**
**Dietrich-Bonhoeffer-Strasse 13**
**O-1055 Berlin(DE)**
Erfinder: **Klebsch, Hans-Joachim,Dr.**
**Rudolf-Reusch-Strasse 50**
**O-1156 Berlin(DE)**
Erfinder: **Ott, Jutta, Dr.**
**Leninplatz 27**
**O-1017 Berlin(DE)**
Erfinder: **Warmuth, Ralf**
**Friedrichshagener Strasse 49**
**O-1170 Berlin(DE)**
Erfinder: **Poppei, Marianne, Dr.**
**Prenzlauer Promenade 165 a**
**O-1100 Berlin(DE)**
Erfinder: **Oehme, Peter, Prof. Dr.**
**Hubertusstrasse 43**
**O-1409 Mühlenbeck/Summt(DE)**
Erfinder: **Hecht, Karl, Prof. Dr.**
**Schützenstr. 13**
**O-1183 Berlin(DE)**
Erfinder: **Becher, Gunther, Dr.**
**Zingster Strasse 36**
**O-1095 Berlin(DE)**
Erfinder: **Krause, Winfried, Dr.**
**Schulze-Boysen-Strasse 33**
**O-1130 Berlin(DE)**
Erfinder: **Bienert, Michael, Dr.**
**Parkstrasse 14**
**O-1114 Berlin(DE)**
Erfinder: **Granitza, Dörthe**
**Chopinstrasse 1**
**O-1120 Berlin(DE)**
Erfinder: **Wachtel, Elena**
**An der Kolonnade 1**
**O-1080 Berlin(DE)**

(74) Vertreter: **Hansen, Bernd, Dr. Dipl.-Chem. et al**
**Hoffmann, Eitle & Partner Patent- und Rechtsanwälte Arabellastrasse 4 Postfach 81 04 20**
**W-8000 München 81(DE)**

(54) **Diaminosäurederivate und pharmazeutische Zusammensetzungen.**

(57) Die Erfindung betrifft Diaminosäurederivate der Formel I

$$R^2HN-CH-CONR^3R^4$$
$$|$$
$$(CH_2)_n \qquad\qquad I,$$
$$|$$
$$NHR^1$$

EP 0 490 379 A2

worin n gleich 2 bis 4, vorzugsweise 3 und 4, ist, $R^1$ und $R^2$ unabhängig voneinander Wasserstoff oder Acylreste, insbesondere Acetyl-, Propionyl- oder tert. Pentanoyl-, bedeuten, $R^3$ und $R^4$ unabhängig voneinander Alkyl oder Aralkyl, vorzusgweise mit 1 bis 10 Kohlenstoffatomen, bedeuten, oder $R^3$ und $R^4$ gemeinsam mit dem Stickstoffatom, an dem sie gebunden sind, einen ggf. substituierten heterocyclischen Ring bilden, der auch weitere Heteroatome enthalten kann, und deren pharmazeutisch verträgliche Salze, pharmazeutische Zusammensetzungen, die diese Diaminosäurederivate enthalten und die Verwendung dieser Diaminosäurederivate zur Herstellung von Arzneimitteln zur Behandlung verschiedener Krankheiten, insbesondere für die Prophylaxe und Therapie von durch chronischen Streß bedingten funktionellen und vegetativen Störungen und zur Verbesserung der Adaptionsfähigkeit gegenüber chronischen Stressor-Einflüssen. Als Stressoren wirken Dauerbelastung durch Immobilisation, Konfliktsituationen, traumatische Faktoren, Alkohol und Suchtmittel bzw. deren Entzug.

Weiterhin werden die erfindungsgemäßen Diaminosäurederivate für die Herstellung eines Antiasthmatikums für die Therapie von allergisch bedingten Störungen der Atemwege verwendet.

Die Erfindung betrifft Diaminosäurederivate, pharmazeutische Zusammensetzungen, die diese Diaminosäurederivate enthalten und die Verwendung dieser Diaminosäurederivate zur Herstellung von Arzneimitteln zur Behandlung verschiedener Krankheiten, insbesondere zur parenteralen und oralen Prophylaxe und Therapie von durch chronischen Streß bedingten funktionellen Störungen sowie zur Therapie von allergisch bedingten funktionellen Störungen der Atemwege.

Die Diaminosäurederivate gemäß der vorliegenden Erfindung werden für die Herstellung von Arzneimitteln zur therapeutischen Behandlung des menschlichen und tierischen Körpers verwendet.

Es ist bekannt, daß Substanz P, ein Neuropeptid mit der Struktur Arg-Pro-Lys-Pro-Gln-Gln-Phe-Phe-Gly-Leu-MetNH$_2$,zur Therapie und Prävention streß- und neurosebedingter Schlafstörungen erfolgreich verwendet werden kann (DD 207 668). Die gleichen Effekte wurden für die Normalisierung der streßinduzierten Hypertension und von streßbedingt gestörten Lern- und Gedächtnisprozessen gefunden (DD 229 593).

Dieses Undekapeptid weist allerdings eine geringe Stabilität auf. Ein Nachteil solcher langkettigen Peptide ist auch ihre aufwendige und kostenintensive Herstellung.

Es gibt Untersuchungen, diese Nachteile durch eine Verkürzung der Sequenz des Undekapeptides zu vermindern. So konnte gezeigt werden, daß N-terminale Teilsequenzen, wie Substanz P-nona-, -hepta- und -tetrapeptide, insbesondere Arg-Pro-Lys-Pro-R (R = OH, NH$_2$) bei intraperitonealer Gabe äquimolarer Mengen eine gleiche und bessere Wirksamkeit aufweisen (DD 229 593 und Pharmazie 41, 1986, 799).

Die ausschließlich parenterale Anwendbarkeit, die kostenaufwendige Herstellung sowie die ebenfalls unzureichende Stabilität auch dieser Teilsequenzen sind jedoch für eine klinische Anwendung hinderlich.

Sowohl Substanz P als auch die bekannten Teilsequenzen zeigen keine orale Wirksamkeit, wodurch die Anwendbarkeit eingeschränkt ist und die Therapie kostenintensiv wird.

Weiterhin sind Peptide bekannt, die als Teilsequenz Lys-Pro enthalten und blutdrucksenkende ( GB 21 63 166 ) und wundheilungsfördernde ( EP 165 492 ) Wirkungen aufweisen, sowie zur Behandlung von degenerativen Erkrankungen verwendet werden ( EP 124 317 ). Die bisher beschriebenen Verwendungsweisen beziehen sich jedoch nicht auf die Behandlung von streßbedingten funktionellen und vegetativen Störungen.

Auch eine antiasthmatische Wirkung von Substanz P oder entsprechender Teilsequenzen, die eine Behandlung allergisch bedingter Atemwegserkrankungen gestatten würde, wurde bisher nicht beschrieben. Aufgrund der Lokalisierung im Respirationstrakt scheint Substanz P hier eine besondere Rolle zu spielen (Uddmann, R. et al. Am. Rev. Respir. Dis. 136, S. 3-8, 1987).

Untersuchungen mit Substanz P an isolierten Atemwegspräparaten, in in-vivo-Untersuchungen am narkotisierten Meerschweinchen oder die inhalative Verabreichung von Substanz P beim Menschen belegen eine bronchokonstriktorische Wirkung, die auf eine proasthmatische Wirkung des Undekapeptids schließen läßt (Schreiber, J. et al., Biomed. Biochim. Acta 47, S. 93-94, 1988; Joost, G. a. a. O.).

Untersuchungen der N-terminalen Teilsequenz SP 1-4 des Undekapeptides an in-vitro- und in-vivo-Asthmamodellen belegen, daß diese Teilsequenz keine antiasthmatische Wirkung aufweist, sowohl nach einmaliger als auch mehrmaliger Applikation.

Bisher ist die Therapie des Bronchialasthmas überwiegend symptomatisch orientiert. In den letzten Jahren wurden mehrere neue Antiasthmatika (z. B. Dinatriumchromoglykat, Ketotifen, Nedocromil) eingeführt. Die Applikation dieser Wirkstoffe erfolgt in Form von Sprays, Pulvern oder Tabletten. Sprays sind im akuten Fall Mittel der Wahl. Für eine Dauertherapie sind sie weniger geeignet, da sie die Schleimhäute angreifen.

Es gibt auch einige qualitativ neue therapeutische Ansätze.

So wurden z.B. Hydroxylaurophonon-Verbindungen (DD 235 450) beschrieben, die jedoch sehr schwer löslich sind. In Wasser sind diese Verbindungen sogar unlöslich, was die Resorption im Organismus sehr erschwert, so daß beispielsweise bei gleichem Applikationsregime sehr unterschiedliche Serumkonzentrationen gefunden wurden. Es ist auch bisher nicht gelungen, Dosis-Wirkungs-Kurven hinsichtlich der Serumkonzentration aufzustellen.

Beschrieben wurden auch Phthalozinonderivate. Hierbei handelt es sich um langanhaltend antiasthmatisch wirkende Verbindungen, die ihre maximale Wirkung erst nach Vorbehandlung über drei Wochen erreichen und somit nicht für akute Asthmaanfälle geeignet sind. Auch haben diese Substanzen eine Reihe von Nebenwirkungen.

Essentiell für alle Asthmaformen ist die bronchiale Hyperreaktivität. Bekannt ist, daß bei der Entstehung und Perpetuierung der bronchialen Hyperreaktivität und des allergischen Asthmas peptiderge Mechanismen beteiligt sind (Nieber, K. et al. Int. Arch. Allergy Appl. Immunol. 505, 1991; Regoli, D. Am. Rev. Respir. Dis. 136, S. 35-39, 1987; Barnes, P. J., Am. Rev. Respir. Dis. 136, S. 77-83, 1987; Joost, G. et al., Thoray 42, S. 779-783, 1987).

Gegenstand der Erfindung sind Diaminosäurederivate der Formel I

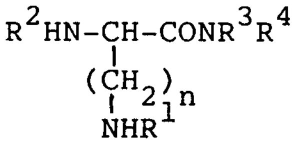

I,

worin n gleich 2 bis 4, vorzugsweise 3 und 4 ist, $R^1$ und $R^2$ unabhängig voneinander Wasserstoff oder Acylreste, insbesondere Acetyl-, Propionyl- oder tert. Pentanoyl-, bedeuten, $R^3$ und $R^4$ unabhängig voneinander Alkyl oder Aralkyl, vorzugsweise mit 1 bis 10 Kohlenstoffatomen, bedeuten, oder $R^3$ und $R^4$ gemeinsam mit dem Stickstoffatom, an dem sie gebunden sind, einen ggf. substituierten heterocyclischen Ring bilden, der auch weitere Heteroatome enthalten kann, und deren pharmazeutisch verträgliche Salze, pharmazeutische Zusammensetzungen, die Diaminosäurederivate der Formel I oder deren pharmazeutisch verträgliche Salze enthalten und die Verwendung von Diaminosäurederivaten der Formel I oder deren pharmazeutisch verträglicher Salze zur Herstellung eines Arzneimittels zur Behandlung verschiedener Krankheiten.

Es wurde gefunden, daß die erfindungsgemäßen Verbindungen eine hohe Wirksamkeit für die Anpassung an Stressoren und zur Beschleunigung der Regenerierung nach Streßschädigungen aufweisen und als Antistreßmittel sowohl parenteral als auch oral anwendbar sind.

Die Verbindungen der Formel I zeigen eine hohe Wirksamkeit bei ihrem Einsatz zur Vermeidung bzw. Normalisierung von funktionellen Störungen im peripheren, vegetativen und zentralen Nervensystem sowie Störungen des Biorhythmus, die durch langzeitige Streßbelastung verursacht werden.

Als Stressoren wirken Dauerbelastungen durch Immobilisation, Konfliktsituationen, traumatische Faktoren sowie Alkohol und Suchtmittel bzw. deren Entzug.

Außerdem wurde gefunden, daß die erfindungsgemäßen Verbindungen eine antibronchokonstriktorische Wirksamkeit aufweisen.

Ist die Bronchokonstriktion allergisch bedingt, zeigen die erfindungsgemäßen Verbindungen eine hohe antiasthmatische Wirksamkeit und eine hohe Abbaustabilität.

Die Verbindungen der Formel I sind aufgrund ihrer Stabilität sowohl oral als auch parenteral zur Behandlung von allergisch bedingten Atemwegserkrankungen anwendbar.

Bevorzugt werden Verbindungen der Formel I eingesetzt, worin $R^3$ und $R^4$ gemeinsam mit dem Stickstoffatom, an dem sie gebunden sind, einen heterocyclischen Ring bilden, der substituiert sein und/oder weitere Heteroatome, wie z.B. Sauerstoff oder Schwefel, enthalten kann. Als Substituenten sind Carboxylgruppen besonders geeignet.

Vorzugsweise bilden $R^3$ und $R^4$ gemeinsam mit dem Stickstoffatom, an dem sie gebunden sind, einen fünf- oder sechsgliedrigen heterocyclischen Ring. Der fünfgliedrige heterocyclische Ring kann vorteilhafterweise eine Carboxylgruppe als Substituent und/oder Schwefel als weiteres Heteroatom enthalten. Der sechsgliedrige heterocyclische Ring kann als weiteres Heteroatom bevorzugt Sauerstoff enthalten.

Eine Auswahl der als Wirkstoff bevorzugt eingesetzten Verbindungen der Formel I zeigt die Tabelle 1.

Eine besonders hohe Wirksamkeit zeigen Verbindungen der Formel I, worin n gleich 4, $R^1$ und $R^2$ jeweils Wasserstoff und $-NR^3R^4$ Prolin, Pyrrolidin oder Morpholin bedeuten, sowie deren $N^{\epsilon}$ -Acetyl-Lysin-Derivate.

Durch die Acetylierung wird die Abbaustabilität der Verbindungen der Formel I weiter erhöht.

Die Herstellung der Verbindungen der Formel I bzw. deren Salze erfolgt nach bekannten Methoden der Peptidchemie, wie sie z. B. in Houben-Weyl "Methoden der Organischen Chemie", Bd. 15, ausführlich beschrieben sind.

Gemäß der Erfindung können die Verbindungen der Formel I als freie Verbindungen, oder in Form ihrer physiologisch verträglichen Salze, z. B. von Mineralsäuren (Hydrochloride, Sulfate u. s. w.) oder als Salze von organischen Säuren, insbesondere von Dicarbonsäuren (z. B. Fumarate), angewendet werden.

Die erfindungsgemäßen Verbindungen werden im allgemeinen auf oralem Wege verabreicht. Eine parenterale oder intravenöse Applikation ist ebenfalls möglich. Die pharmazeutischen Zusammensetzungen bestehen aus einer oder mehreren der erfindungsgemäßen Verbindungen der Formel I oder enthalten eine oder mehrere der Verbindungen der Formel I neben nichttoxischen, inerten und pharmazeutisch geeigneten Trägerstoffen und Zusätzen, wie Stabilisatoren, Füllstoffen, Bindemitteln, Adsorptions- und Gleitmitteln.

Bevorzugte pharmazeutische Zusammensetzungen sind Lösungen, Sprays, Emulsionen, Suspensionen, Tabletten, Dragees und Granulate.

Der Gehalt der wirksamen Verbindungen in den genannten pharmazeutischen Zusammensetzungen beträgt 2 bis 500 mg pro Dosiereinheit. Für die orale Anwendung ist der Wirkstoff in einer Dosierung von 20 bis 200 mg pro Dosiereinheit enthalten; für die parenterale Verabreichung in einer Dosierung von 2 bis 20 mg.

Die Herstellung der pharmazeutischen Zusammensetzungen erfolgt in üblicher Weise und nach bekannten Methoden, z. B. durch Mischen der Wirkstoffe mit Träger- und/oder Zusatzstoffen, bzw. Lösen der Wirkstoffe in geeigneten Flüssigkeiten.

Die erfindungsgemäßen Verbindungen und pharmazeutischen Zusammensetzungen wurden auf ihre adaptogene Wirkung bei intraperitonealer und oraler Gabe an Ratten geprüft und z. T. mit Substanz P verglichen.

Folgende Tests wurden durchgeführt:

a) Normalisierung der durch Immobilisationsstreß bedingten Hypertonie

b) Aufhebung der an den gleichen Tieren bei Dauerstreß herausgebildeten "endogenen Opioidabhängigkeit"

c) Restauration bzw. Erhaltung der Lern- und Gedächtnisleistungen nach streßinduzierter Störung (avoidance learning) bzw. der durch Elektroschock verursachten Amnesie

d) Heilung chronisch-experimenteller Neurosen

e) Beseitigung der durch Konflikstreß induzierten Störungen des Schlafprofils

f) Verhinderung der Folgen einer pränatalen Alkoholintoxikation

g) Reduzierung der Entzugserscheinungen nach Zwangsalkoholisierung

Die Ergebnisse der Tests sind in den Beispielen 5 bis 12, der Abbildung 1 und in den Tabellen 2 bis 9 dargestellt.

Es wurde gefunden, daß Verbindungen der Formel I, wie z. B. das Dipeptid Lys-Pro, bei intraperitonealer Gabe in gleicher Konzentration ( $2x10^{-7}$ Mol/kg ) wirksam sind wie Substanz P 1-11. Die Verbindungen der Formel I zeigen im Gegensatz zu Substanz P 1-11 und zu dem N-terminalen Tetrapeptid SP 1-4 eine orale Wirksamkeit bei der Normalisierung des streßbedingten Hypertonus.

Während für eine orale Wirksamkeit von Lys-Pro noch eine 200fache Dosierung gegenüber der i.p. Dosis erforderlich ist, genügen z. B. beim Pyrrolidid und Morpholid des Lysins sowie bei deren $N^{\epsilon}$ -Acylderivaten bereits 5- bis 20-fache Gaben für eine Normalisierung des streßbedingten Hypertonus und für die Aufhebung der bei Dauerstreß herausgebildeten "endogenen Opioidabhängigkeit" .

Die orale Dosierung ($4x10^{-6}$ Mol/kg) der erfindungsgemäßen niedermolekularen Verbindungen stellt gegenüber dem perenteralen Einsatz langkettiger und oral unwirksamer Substanz P-Peptide einen erheblichen Vorteil dar.

Die erfindungsgemäßen Antistreßmittel sind abbaustabil und weisen eine selektivere Wirsamkeit bei der Vermeidung und Normalisierung von streßbedingten funktionellen Störungen auf als z. B. ZNS-Pharmaka.

Ein Vorteil der erfindungsgemäßen Mittel besteht darin, daß sie sowohl parenteral als auch oral anwendbar sind.

Der Einsatz dieser Antistreßmittel gewährleistet damit eine kostengünstige Therapie und Prophylaxe streßbedingter Dysregulationen.

Anwendung finden die Verbindungen der Formel I in pharmazeutischen Zusammensetzungen für die Humanmedizin zur parenteralen und oralen Prophylaxe und Therapie von durch chronische Stressor-Einwirkung bedingten Störungen in der Regulation peripherer, vegetativer und zentraler Funktionen, sowie des Biorhythmus. Dies gilt auch in der Gravidität, um eine Übertragung der Streßschäden auf die Nachkommen zu verhindern.

Zu den genannten Störungen gehören hormonelle Dysregulationen, Störungen von Herz-Kreislauf-Funktionen, von Schlaf, Verhalten, Lern- und Gedächtnisleistungen.

Außerdem sind die Verbindungen der Formel I zur Verbesserung der Adaptationsfähigkeit gegenüber Stressor-Einflüssen, insbesondere Immobilisation, Konflikten, Traumata, Alkohol und Suchtmitteln sowie deren Entzug einsetzbar.

In der Tierhaltung können diese Verbindungen und pharmazeutischen Zusammensetzungen zur Vermeidung von streßbedingten Schäden, insbesondere bei der Massentierhaltung und beim Transport eingesetzt werden.

Die erfindungsgemäßen Verbindungen wurden außerdem auf ihre antiasthmatsiche Wirkung in-vitro an isolierten Trachea- und Lungenparenchympräparaten des Meerschweinchens und in-vivo am Asthmamodell des narkotisierten Meerschweinchens geprüft. Am in-vivo-Modell erfolgte die Substanzverabreichung oral und intravenös. Allergische Reaktionen wurden durch Ovalbumin induziert.

Folgende Tests wurden durchgeführt:

a) Wirksamkeit im allergischen Asthmamodell des narkotisierten Meerschweinchens

b) Einfluß auf die durch Acetylcholin (ACh) induzierten Kontraktionen an Trachea- und Lungenparenchympräparaten von Ovalbumin-sensibilisierten und unbehandelten Tieren

c) Einfluß auf die durch Ovalbumin induzierten Kontraktionen an Trachea- und Lungenparenchympräparaten von Ovalbuminsensibilisierten Tieren

d) Einfluß auf die elektrisch evozierte ACh-Freisetzung aus Tracheaschnitten von Ovalbumin-sensibilisierten Tieren

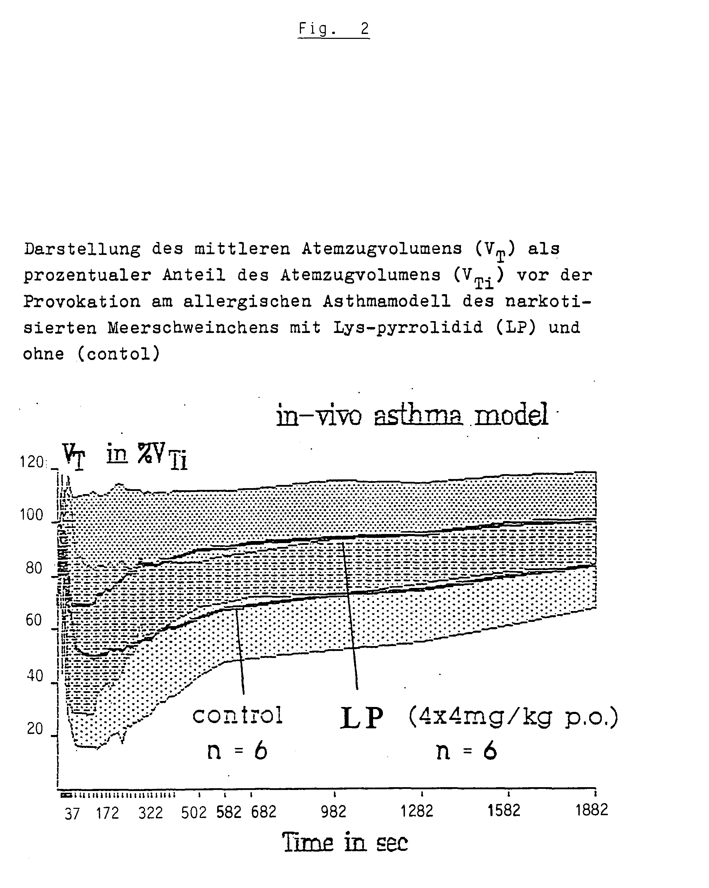

Die Ergebnisse der Tests sind in den Beispielen 13 bis 16, der Abbildung 2 und in den Tabellen 10 bis 12 dargestellt.

Es wurde gefunden, daß Verbindungen der Formel I, wie z. B. Lys-pyrrolidid, Lys-morpholid und das Dipeptid Lys-Pro, nach oraler Gabe (4x4 mg/kg) eine signifikante Hemmwirkung auf den allergischen Bronchospasmus am narkotisierten Meerschweinchen haben. Die Verbindungen haben keine Wirkung auf den durch Arachidonsäure ausgelösten Bronchospasmus des Meerschweinchens.
In-vitro, an isolierten Trachea- und Lungenparenchymstreifen von sensiblisierten Meerschweinchen hemmen die erfindungsgemäßen Verbindungen die durch ACh bzw. Ovalbumin ausgelösten Kontraktionen. An Präparaten nicht sensibilisierter Tiere waren die Verbindungen wirkungslos. Weiterhin wird gezeigt, daß die Verbindungen die elektrisch evozierte ACh-Freisetzung aus Tracheaschnitten von Ovalbumin-sensiblisierten Meerschweinchen hemmen.

Die erfindungsgemäßen Verbindungen wirken protektiv und weisen eine selektive Wirksamkeit bei der Beeinflussung allergisch bedingter Atemwegserkrankungen auf. Sie sind oral applizierbar und abbaustabil und daher für eine Dauertherapie geeignet, zumal auch kaum Nebenwirkungen nachgewiesen werden konnten.

Im folgenden werden Synthesen, Zubereitungen, Methoden und Ergebnisse der biologischen Prüfung sowie Struktur-Wirkungs-Beziehungen durch Beispiele belegt.

Beispiele

Beispiel 1 *Synthese der Diaminosäureamide*

10 mmol der $\alpha,\omega$-N-geschützten Diaminosäure, zum Beispiel Boc-Lys(Boc), werden in 30 ml $CH_2Cl_2$ mit 11 mmol N-Methylmorpholin in Lösung gebracht, bei -15 °C mit 10 mmol Cl-COOiso-Butyl 15 min aktiviert, mit 10 bis 20 mmol Amin unter Rühren versetzt und 30 min bei -10 bis -15 °C, 30 min bei 0 °C und 3 h bei 20 °C gerührt. Nach Entfernen des $CH_2Cl_2$ wird in Ethylacetat aufgenommen, mit 5proz. $KHSO_4$-Lösung, 5proz. $HaHCO_3$-Lösung, gesättigter NaCl-Lösung geschüttelt, über $Na_2SO_4$ getrocknet und zur Trockne gebracht. Der Rückstand wird mit 100 mmol HCl in Ethylacetat (3,5 m Lösung) je 10 mmol Boc-Schutzgruppe 1 h bei 20 °C und nach Zugabe von 10 ml Wasser noch 30 min gerührt.
Die wäßrige Phase wird noch 3 x mit Ethylacetat extrahiert und bei 40 °C am Vakuumrotationsverdampfer getrocknet. Der Rückstand wird mehrmals mit absol. Ethanol aufgenommen und eingeengt. Am Ende werden die Hydrochloride festgerieben. Die freien Basen werden erhalten, indem man in 10 ml Wasser löst, mit $NH_3$-Lösung auf pH = 10 einstellt, das Wasser im Vakuum abdestilliert, den Rückstand mit trocknem Chloroform extrahiert und den Extrakt zur Trockne bringt.

Beispiel 2 *Gewinnung der acetylierten Derivate durch nachträgliche Acetylierung*

Ausgehend von $N^{\alpha}$-Boc und $N^{\omega}$- Z- geschützten Diaminosäuren, z. B. Boc-Lys(Z), wird wie in Beispiel 1 verfahren. Die selektive Abspaltung der Z-Schutzgruppe erfolgt durch katalytische Hydrierung von 10 mmol des geschützten Diaminosäureamids in 10 ml Wasser/Eisessig/Methanol 1:1:8 mit 2 mg Pd-Kohle über 6 h bei Raumtemperatur im $H_2$-Strom. Das Filtrat wird im Vakuum zur Trockne gebracht.
Der jeweilige Cl-freie Rückstand wird in 10 ml Aceton/Wasser (Verhältnis 1:1) pro 10 mmol Base gelöst und mit 11 mmol AcONB (N-Acetoxy-norbornendicarboximid) versetzt.
Nach vollständiger Umsetzung (DC-Kontrolle) wird das Lösungsmittel im Vakuum abdestilliert, der Rückstand in Ethylacetat aufgenommen und so, wie in Beispiel 1 für die geschützten Amide beschrieben, aufgearbeitet.
Die Diacetyl-Verbindungen werden als sirupartiger Rückstand getrocknet und weiter eingesetzt.
Bei der Monoacetylierung werden die Boc-Schutzgruppe wie oben beschrieben entfernt und Hydrochloride bzw. Basen analog isoliert.

Beispiel 3 *Alternative Synthese von $N^{\epsilon}$ -Acyl-lysinamiden*

10 mmol $N^{\epsilon}$ -Acetyl-lysin werden mit Di-tert.butyldicarbonat in bekannter Weise in $N^{\alpha}$-Boc, $N^{\epsilon}$-Acetyl-

lysin und dann wie in Beispiel 1 beschrieben, in das geschützte Amid sowie nach Abspaltung der Boc-Schutzgruppe analog in das Monohydrochlorid oder das freie Monoacetyl-Derivat überführt.

Beispiel 4 *Gewinnung der Fumarate*

Die nach Beispiel 1 und 2 erhaltenen freien und $N^{\omega}$-acylierten Diaminosäureamide (10 mmol) werden unter Zusatz eines Äquivalents Fumarsäure in etwas Wasser und 10 ml Ethanol gelöst.
Nach kurzem Erwärmen wird das Lösungsmittel entfernt und der Rückstand in Ethanol oder Ethylacetat bzw. nach Fällung aus Ethanol mit Aceton festgerieben.
Die nach Beispiel 1 bis 4 gewonnenen Verbindungen sind in Tabelle 1 zusammengestellt.

**Tabelle 1:** $R^2$-NH-CH-CON$R^3R^4$ · mX

$$R^2\text{-NH-}\underset{\underset{\displaystyle NHR^1}{|}}{\underset{\displaystyle (CH_2)_n}{\underset{|}{CH}}}\text{-CONR}^3\text{R}^4 \cdot mX$$

| Nr. | n | $R^1$ | $R^2$ | $NR^3R^4$ | m | X | Beschreibung |
|---|---|---|---|---|---|---|---|
| 1 | 4 | H | H | $-NH(CH_2CH_3)_2$ | 1 | Fumarsäure (Hydrat) | Fp. 173-5 °C |
| 2 | 4 | H(L-Lys) | H | 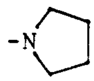 | 2 | HCl | Fp. 245-4 °C<br>$[\alpha]^{20}_D$=-8,44°<br>c=5 in 5n HCl |
| | | | | | 1 | Fumarsäure (Hydrat) | Fp. 28-101 °C |
| 3 | 4 | H(D-Lys) | H | 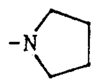 | 2 | HCl | Fp. 245-6 °C<br>$[\alpha]^{20}_D$= 8,40°<br>c=5 in 5n HCl |
| 4 | 4 | H | $COCH_3$ | 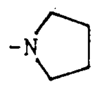 | 1 | HOAc | Öl, $M^+$: 241 |
| 5 | 4 | $COCH_3$ | H | 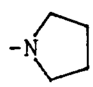 | 1 | HCl | Öl, $M^+$: 241 |
| | | | | | 0,5 | Furmarsäure (Hydrat) | Fp. 100-105 °C |
| 6 | 4 | $COCH_2CH_3$ | H | 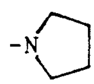 | 1 | HCl | amorph,<br>hygroskopisch<br>$M^+$: 255 |
| 7 | 4 | $COCH_3$ | $COCH_3$ | 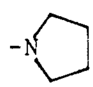 | | | Öl, $M^+$: 283 |
| 8 | 4 | $COC(CH_3)_3$ | $COC(CH_3)_3$ | 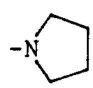 | | | Fp. 148-9 °C,<br>$M^+$: 367 |
| 9 | 4 | H | H | 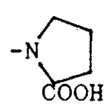 | 2 | HCl | amorph |

| | | | | | | | |
|---|---|---|---|---|---|---|---|
| 10 | 4 | H | $COCH_3$ | 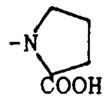 | 1 | HOAc | hygroskopisch<br>$M^+$: 285 |
| 11 | 4 | $COCH_3$ | $COCH_3$ | 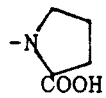 | | | Fp. 185-90 °C |
| 12 | 4 | H | H | 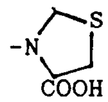 | 2 | HCl | amorph,<br>hygroskopisch |
| 13 | 4 | $COCH_3$ | $COCH_3$ | 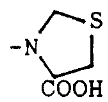 | | | amorph,<br>hygroskopisch |
| 14 | 4 | H | H | 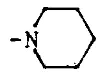 | 2 | HCl | hygroskopisch<br>$M^+$: 213 |
| 15 | 4 | $COCH_3$ | $COCH_3$ | 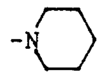 | | | Öl, $M^+$: 297 |
| 16 | 4 | H | H | 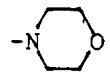 | 2 | HCl | hygroskopisch<br>$M^+$: 215 |
| 17 | 4 | $COCH_3$ | H | 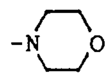 | 1 | HCl | Öl, $M^+$: 257 |
| 18 | 4 | $COCH_3$ | $COCH_3$ | 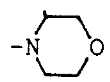 | | | Öl, $M^+$: 299 |
| 19 | 3 | H | H | 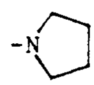 | 2 | HCl | Fp. 95-100 °C |
| 20 | 3 | H | H | 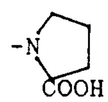 | 2 | HCl | amorph,<br>hygroskopisch |
| 21 | 3 | $COCH_3$ | $COCH_3$ | 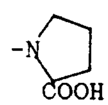 | | | amorph<br>hygroskopisch<br>$M^+$: 313 |
| 22 | 2 | H | H | 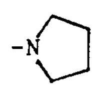 | 2 | HCl | Fp. 134-8 °C |

Beispiel 5 *Wirkung auf die streßbedingte Hypertonie an der normotonen Ratte* (vgl. DD 229 593)

Männliche Wistar-Ratten werden bis zu 42 Tagen einer intermittierenden Immobilisation nach einem stochastischen Muster ausgesetzt. Während dieser Zeit entwickelt sich eine streßbedingte Hypertonie. Die Blutdruckmessung erfolgte unblutig über eine Schwanzmanschette nicht früher als 4 Stunden nach der Immobilisationsphase, um akute Streßeffekte auszuschalten. Die Blutdruckwerte des Vorbeobachtungsabschnittes werden in der 4. Woche ermittelt. Die Substanzapplikation erfolgte in der 5. Woche täglich über 4 Tage. Die Substanzeffekte auf den Blutdruck werden 24 Stunden nach der jeweiligen vorausgehenden

Substanzgabe erfaßt.
Die in Tabelle 2 dargestellten Blutdruckwerte repräsentieren den Medianwert der gesamten Versuchsgruppe im jeweiligen Versuchsabschnitt. Die Substanzen Nr. 6 und 9 führen auch bei oraler Gabe den streßinduzierten Blutdruckanstieg besonders deutlich auf die Normalwerte zurück.

Beispiel 6 *Wirkung auf die "endogene Opioidabhängigkeit" (gut dependence)*

Isolierte Dünndarm(Ileum)-Abschnitte morphinabhängiger Ratten kontrahieren unter Einwirkung des Opiatantagonisten Naloxon (sog. "gut dependence"), während der Darm nichtopiatabhängiger Tiere unter Naloxon seinen Tonus nicht ändert (vergl. A. R. Gintzler, Brain Res. 1980, 182, 224). Überraschenderweise wurde gefunden, daß chronischer Streß den gleichen Effekt wie eine Morphinbehandlung auslöst, was als Herausbildung einer physischen Abhängigkeit gegenüber endogenen Opioiden gewertet werden kann, weil diese unter Stressoreinwirkung in stark erhöhtem Maße freigesetzt werden. Die erfindungsgemäßen Substanzen mit Antistreß-Wirksamkeit führten die durch Naloxon induzierbare pathologische Reaktionsweise des Darmes gestreßter Tiere ebenfalls wieder zum Normalzustand zurück (d.h. keine Naloxoninduzierbare Kontraktion). In Tabelle 2 ist in der Spalte "endogene Opioidabhängigkeit" die Wirksamkeit der einzelnen Substanzen dargestellt.

Tabelle 2: Wirksamkeit bei streßbedingter Hypertonie und endogener Opioidabhängigkeit an der Ratte[a)]

| Nr. | Substanz | n | Dosis \|µg/kg\| | Applikation | Blutdruck (mmHg) nach Streß | Streß+Behandlung | "Endogene Opioid-abhängigkeit" b) |
|---|---|---|---|---|---|---|---|
| 0 | Kontrolle c) | 10 | 10 ml $H_2O$ | i.p. und oral | 132-138 | 130-135 | 10/0 |
| 1 | Lys-Pro·2HCl | 5 | 59 | i.p. | 132 | 121 d)e) | 5/0 |
| | | 8 | 11800 | oral | 136 | 120 d)e) | n.t. |
| 2 | Ac-Lys-Pro·HOAc | 9 | 57 | i.p. | 135 | 129 d) | 0/5 |
| 3 | Ac-Lys(Ac)-Pro | 8 | 1214 | oral | 138 | 133 | 5/1 |
| 4 | Lys-$NMe_2$·fumarat | 9 | 65 | i.p. | 135 | 134 | 6/3 |
| 5 | D-Lys-Pyrrolidid·2HCl | 8 | 50 | i.p. | 136 | 137 | 6/2 |
| 6 | Lys-pyrrolidid·2HCl | 6 | 123 | i.p. | 139 | 130 d) | 1/5 |
| | | 9 | 1228 | oral | 135 | 129 d)e) | 2/6 |
| 7 | Orn-pyrrolidid·2HCl | 9 | 65 | i.p. | 135 | 123 d) | 1/8 |
| 8 | Ac-Lys-pyrrolidid·HOAc | 4 | 61 | i.p. | 120 | 118 | n.t. |
| 9 | Lys(Ac)-pyrrolidid·HCl | 6 | 53 | i.p. | 134 | 128 d)e) | 0/5 |
| | | 8 | 1100 | oral | 136 | 126 d)e) | 3/3 |
| | ·fumarat | 6 | 1140 | oral | 133 | 128 d)e) | 5/1 |
| 10 | Ac-Lys(Ac)-pyrrolidid | 6 | 1050 | oral | 140 | 139 | 3/3 |
| 11 | Ac-Lys(Ac)-piperidid | 7 | 47 | i.p. | 135 | 129 d)e) | 4/1 |
| | | 6 | 940 | oral | 137 | 138 | 4/2 |
| 12 | Lys-morpholid·2 HCl | 7 | 125 | i.p. | 140 | 130 d) | 3/4 |
| 13 | Lys(Ac)-morpholid·HOAc | 8 | 96 | i.p. | 139 | 136 | n.t. |
| 14 | Lys($C_2H_5CO$)-pyrrolidid·HOAc | 7 | 94 | i.p. | 140 | 133 d) | 6/1 |

a) Blutdruck vor Streßbehandlung: 120-130 mm Hg; b) Anteil der Tiere, die nach Behandlung positiv/nicht reagierten; c) eine Kontrolle wurde bei jeder Testgruppe (3-4 Substanzen) mitgeführt; d) Signifikanz gegenüber der Vorbeobachtung unter Streß ($p < 0,05$); e) Signifikanz gegenüber der Kontrollgruppe im jeweiligen Versuchsabschnitt ($p < 0,05$)
n.t. nicht getestet

Beispiel 7 *Behandlung streßinduzierter Schlafstörungen*

Männliche adulte Ratten werden chronisch gestreßt, indem sie einer vier Wochen dauernden Konfliksituation ausgesetzt wurden, was chronische Schlafstörungen zur Folge hatte. Bereits vor Streßbelastung waren den Tieren Elektroden zur Registrierung des EEG und des EMG implantiert worden. Substanz P

(täglich 250 µg/kg i.p.) und Lys-Pro (täglich 54 µg/kg i.p.), Lys-pyrrolidid (täglich 4 mg/mg oral) und $N^{\epsilon}$ -Acetyl-Lys-pyrrolidid (täglich 4 mg/kg oral) wurden an den letzten vier Tagen der Streßbelastung appliziert.
Die schlafpolygraphischen Untersuchungen erfolgten im Aktivitätsminimum des zirkadian-rhythmischen Verlaufes der Ratte (in Anlenhung an Rechtschaffen und Kales 1969). Untersuchte Parameter und Ergebnisse sind in Tabelle 3 dargestellt. Sie belegen, daß Lys-Pro ebenso wie SP 1-11 nach i.p. Gabe die streßinduzierten Abweichungen in nahezu allen Parametern nach viertägiger Behandlung wieder zur Norm zurückführt.
Orale Gaben von Lys-pyrrolidid und $N^{\epsilon}$ -Acetyl-Lys-pyrrolidid zeigen einen gleichartigen Effekt.

Tabelle 3: Dauer der Wach-Schlaf-Stadien in % (Mittelwerte, n=12)

| Schlaf-Stadium | Kontrollen | nach Streß[a)] ($p < 0.01$) | nach Streß[b)] + SP 1-11 i.p. | nach Streß[b)] + Lys-Pro i.p. | Kontrollen | nach Streß[a)] $p < 0,01$ | nach Streß[a)] + Lys(Ac)Prd oral | nach Streß[a)] $p < 0,01$ | nach Streß[b)] + LysPrd oral |
|---|---|---|---|---|---|---|---|---|---|
| I aktives Wachsein | 12,8 | 23,9 | 10,7 | 10,2 | 20,8 | 51,6 | 36,1 | 44,2 | 30,4 |
| II relaxiertes Wachsein | 3,2 | 11,0 | 3,9 | 4,7 | 16,9 | 16,8 | 17,3 | 24,0 | 10,0 |
| III Oberflächlicher Schlaf | 12,4 | 21,7 | 15,2 | 13,4 | 18,6 | 9,4 | 14,9 | 11,5 | 16,3 |
| IV Tiefschlaf | 56,8 | 36,3 | 59,3 | 60,4 | 33,8 | 19,3 | 25,2 | 17,3 | 35,2 |
| V Paradoxer Schlaf | 14,7 | 7,1 | 10,7 | 11,3 | 9,9 | 2,9 | 6,5 | 2,3 | 8,0 |

a) 24 h nach Absetzen des Streß ; b) 24 h nach Verabfolgung der 4. Tagesdosis; Prd = pyrrolidid.

Beispiel 8 *Lern- und Gedächtnisstörung bei experimentell neurotischen Ratten*

Die Untersuchungen erfolgten mittels der Methode "avoidance learning" an adulten männlichen Wistar-Ratten, bei denen vorher eine experimentelle Neurose mittels "Konflikt durch Unbestimmtheit" über einen Zeitraum von 5 Wochen induziert worden war. Die Dosierung der Peptide erfolgte wie in Beispiel 7.

Die Ergebnisse in Tabelle 4 enthalten den Prozentsatz richtiger Antworten an vier aufeinanderfolgenden Trainingstagen. Sie belegen, daß Lys-Pro die mit der experimentellen Neurose in Zusammenhang stehenden Lernstörungen ebenso vollständig behebt wie SP 1-11 .

Tabelle 4

| Tag | Kontrolle n = 30 | Exp. Neurose n = 30 | Exp. Neurose + SP 1-11 n = 30 | Exp. Neurose + Lys-Pro n = 30 |
|---|---|---|---|---|
| 1. Tag | 36 % | 0 %* | 36 % | 18 % |
| 2. Tag | 64 % | 19 %* | 89 % | 78 % |
| 3. Tag | 92 % | 34 %* | 96 % | 96 % |
| 4. Tag | 100 % | 46 %* | 100 % | 100 % |

* versus Kontrollen $p < 0{,}01$

Nach Ausbildung der bedingten avoidance-Reaktion wurde mittels eines Retentionstestes (10 bedingte Stimuli ohne Bekräftigung in 30-Sekunden-Intervallen) die Gedächtnisleistung (Retentionsrate) unter gleichen Versuchsbedingungen geprüft.

Den Prozentsatz richtiger Antworten auf die unbekräftigten Stimuli zeigt die nachstehende Tabelle:

| Stimulus | Kontrolle n = 30 | Exp. Neurose n = 30 | Exp. Neurose + SP 1-11 n = 30 | Exp. Neurose + Lys-Pro n = 30 |
|---|---|---|---|---|
| 1 bis 10 | 100 % | 48 - 56 %* | 100 % | 100 % |

* versus Kontrollen $p < 0{,}01$

Beispiel 9 *Wirkung von Lys-pyrrolidid (LP) auf die durch Elektrokonvulsiven Shock (ECS) induzierte*

Die durch ECS induzierte retrograde Amnesie, ein Standardmodell zur Untersuchung nootroper Wirkungen, wurde an adulten männlichen Wistar-Ratten erzeugt.

Lys-pyrrolidid wurde oral (4mg/kg) unmittelbar nach dem ECS verabreicht, um die Wirkung der Substanz auf die Gedächtnisleistung (Verhinderung der Abschwächung der retrograden Amnesie) untersuchen zu können.

Ergebnisse sind in Tabelle 5 und in Abbildung 1 dargestellt. Getestet wurde am Modell der aktiven bedingten Fluchtreaktion (Shuttle Box). Die Tiere mußten lernen, nach Einsetzen von bedingten Reizen (Licht und Ton kombiniert) aus einer dunklen Box in eine beleuchtete Box zu wechseln, bevor ein Fußreiz (unbedingter Reiz) als negative Bekräftigung verabreicht wurde (bedingte Reaktion).

Zur Pzüfung der Retention wurde 24 h nach dem Training ein Relearning durchgeführt, das genau wie das Training mit fünf bedingten Reaktionen gestaltet wurde.

Die Zahl der unbedingten Reaktionen in Trainings- und Relearningsitzungen wurde verglichen und zur Bewertung der Gedächtnisleistung herangezogen (RI):

$$RI = \frac{\text{unbedingte Reaktionen im Training} - \text{unbedingte Reaktionen im Relearning}}{\text{unbedigte Reaktionen im Training}} \times 100\ (\%)$$

Der ECS wurde den Ratten mit Hilfe eines Stimulationsgerätes (Rechteckimpuls; 50 Hz; 30 mA; 250 ms - 1 s) über Ohrklemmen verabreicht. Bei den Kontrolltieren wurde eine Schein-ECS-Behandlung (ECS*) vorgenommen, indem die Ohrklemmen nur kurzfristig angelegt wurden, ohne einen Reiz auszulösen. Durch die

Gabe des ECS unmittelbar nach dem Training wird die Konsolidierung der Gedächtnisspur gestört, was sich in einer verminderten Retentionsleistung widerspiegelt (retrograde Amnesie).
Für diese Versuchsserie wurden drei Gruppen gebildet.

Tabelle 5: Retentionsindices (RI in %) aller Tiere der Gruppen 1 bis 3

| Gruppe | 1 | 2 | 3 |
|---|---|---|---|
| | NaCl<br>ECS*<br><br>n = 13 | NaCl<br>ECS<br><br>n = 13 | LP<br>ECS<br>4 mg/kg<br>n = 21 |
| | 13,3 | - 80,0 | - 80,0 |
| | 36,8 | - 18,8 | - 6,2 |
| | 46,7 | - 11,1 | - 5,9 |
| | 47,2 | - 8,7 | 0 |
| | 50,0 | - 5,0 | 8,3 |
| | 50,0 | 0 | 41,7 |
| | 53,8 | 0 | 42,9 |
| | 57,6 | 20,0 | 42,9 |
| | 58,8 | 24,2 | 50,0 |
| | 64,7 | 27,0 | 51,7 |
| | 66,7 | 41,9 | 53,1 |
| | 77,8 | 45,4 | 60,0 |
| | 77,8 | 73,3 | 62,2 |
| | | | 64,7 |
| | | | 66,7 |
| | | | 72,2 |
| | | | 72,2 |
| | | | 73,0 |
| | | | 75,9 |
| | | | 78,3 |
| | | | 78,9 |
| $\bar{X}$ | 53,8 | 0 | 53,1 |
| U-Test | | p < 0,01 | p < 0,01 |

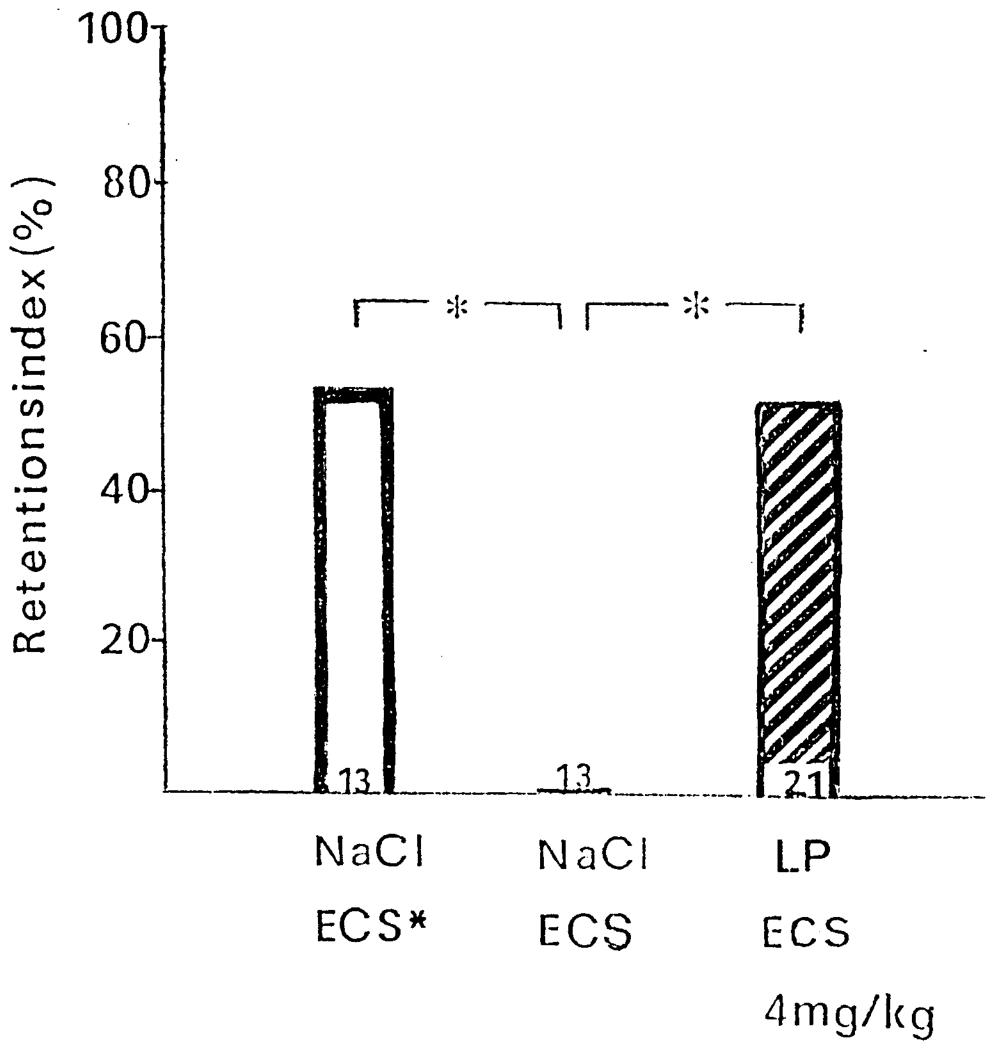


Abbildung 1

Beispiel 10 *Ausdauerbelastung bei experimenteller Neurose*

Nach Ausbildung einer bedingten avoidance-Reaktion an vier aufeinanderfolgenden Tagen wurden im Zeitraum von 45 Minuten 90 unbekräftigte bedingte Stimuli verabreicht, die von den Tieren mittels eines Hebeldruckes zu beantworten waren. Die experimentelle Neurose (chronischer Streß) wurde wie in Beispiel 8 hervorgerufen.
Die durch die experimentelle Neurose verursachte Einschränkung der Ausdauerbelastung wurde durch Lys-Pro gleichermaßen kompensiert wie durch SP 1-11 (Dosierung wie in Beispiel 7).

Die nachstehenden Ergebnisse in Tabelle 6 belegen auch in diesem Test eine sehr gute Wirksamkeit der erfindungsgemäßen Verbindungen.

Tabelle 6: Antworten auf bedingte Stimuli in 5er Gruppen zusammengefaßt (n = 30 je Gruppe)

| Reize | Kontrollen | Exp. Neurose | Exp. Neurose + SP 1-11 | Exp. Neurose + Lys-Pro |
|---|---|---|---|---|
| 5 | 100 % | 56 %* | 100 % | 100 % |
| 10 | " | 51 %* | " | " |
| 15 | " | 48 %* | " | " |
| 20 | " | 45 %* | " | " |
| 25 | " | 51 %* | " | " |
| 30 | " | 48 %* | " | " |
| 35 | " | 47 %* | " | " |
| 40 | " | 48 %* | " | " |
| 45 | " | 47 %* | " | " |
| 50 | " | 43 %* | " | " |
| 55 | " | 54 %* | " | " |
| 60 | " | 40 %* | " | " |
| 65 | " | 45 %* | " | " |
| 70 | " | 29 %* | " | " |
| 75 | " | 45 %* | " | " |
| 80 | " | 23 %* | " | " |

* versus Kontrollen $p < 0,01$

Beispiel 11 *Folgen einer pränatalen Alkoholintoxikation und deren Verhinderung*

Fünf weibliche adulte Ratten wurden mit einem männlichen Tier so lange gemeinsam gehalten, bis morgens im Vaginalausstrich Spermatozyten nachweisbar waren. Von diesem Zeitpunkt an erhielten die weiblichen Tiere zur Deckung des Flüssigkeitsbedarfes 15 %-ige Ethanollösung ad libitum. Ab dem 16.Tag der Trächtigkeit wurde einer Gruppe SP 1-11 in einer Dosis von 250 µg/kg und einer zweiten Gruppe Lys-Pro in einer Dosis von 54 µg/kg i.p. täglich einmal verabreicht. Nach dem Werfen erhielten die Muttertiere Wasser zur Decktung des Flüssigkeitsbedarfes. Die Nachkommen dieser Muttertiere wurden unter normalen Umständen aufgezogen. Im Alter von 3 Monaten wurden verschiedene funktionelle Untersuchungen durchgeführt.

Unbehandelte Tiere wiesen Verhaltens- und Schlafstörungen auf. Die Applikation von SP 1-11 bzw. Lys-Pro in den letzten Trächtigkeitstagen (ab 16.Tag) bis zum Werfen verhinderte diese Störungen (s.Tabelle 7).

Tabelle 7

| Dauer der Wach-Schlaf-Stadien in % | | | | |
|---|---|---|---|---|
| Stadium | Kontrollen n = 45 | Schlafstörungen durch Alkoholintoxikation n = 50 | Schlafstörungen durch Alkoholintoxikation + SP 1-11 n = 42 | Schlafstörungen durch Alkoholintoxikation + Lys-Pro n = 43 |
| I aktives | 20,53 % | 33,03 %* | 19,73 % | 18,04 % |
| II relaxiertes | 1,75 % | 4,24 %* | 4,00 % | 4,90 % |
| III oberflächlicher Schlaf | 7,81 % | 14,14 %* | 9,66 % | 11,14 % |
| IV Tiefschlaf | 60,18 % | 41,60 %* | 55,87 % | 56,36 % |
| V Paradoxer Schlaf | 9,72 % | 7,00 %* | 10,74 % | 9,56 % |
| Schlafindex | 77 % | 64 % | 76 % | 77 % |

* versus Kontrollen $p < 0,01$

Beispiel 12 *Wirkung auf das Entzugsverhalten nach Zwangsalkoholisierung von Ratten*

44 männliche gemischtrassige weiße Ratten wurden durch zwangsweise Verabreichung hoher Alkoholdosen zu physischer Abhängigkeit geführt. Sie erhielten per Schlundsonde 9 Alkoholgaben im Abstand von 12 Stunden. Die mittlere Dosis betrug 5 g/kg KM in einem Volumen von 2 ml/100 g KM.

Die Zwangsalkoholisierung der Ratten und ein Verhaltenstest zur Bewertung der Alkoholentzugssymptome wurden nach der Methode von Majchrovicz und einer Variation dieser Methode zur Verkürzung der Alkoholisierungsphase nach Abrawschikow durchgeführt (Majchrowicz, E., Psychopharmakologia 43, 1975, S. 245-254; Abrawschikow, A. CH., Farmakologia i Toksikologia 46, 1983, S. 94-98).

Es wurden 4 Gruppen gebildet:

Gruppe I: NaCl; Placebo

Gruppe II: 1 x Lys-pyrrolidid (LP) (1 d)
Einmalige Gabe von 4 mg/kg Lys-pyrrolidid oral unmittelbar nach letzter Alkoholgabe.

Gruppe III: 1 x Lys-pyrrolidid (1 h)
Einmalige Gabe von 4 mg/kg Lys-pyrrolidid oral 1 Stunde vor Beginn des 1. Beobachtungstestes zur Bewertung des Entzugsverhaltens (16 Stunde nach Entzug).

Gruppe IV: 4 x Lys-pyrrolidid
Viermalige Gabe von 4 mg/kg Lys-pyrrolidid oral im Abstand von 12 Stunden während der Zwangsalkoholisierung.

Zu allen angegebenen Applikationszeiten erhielten die Tiere der anderen Gruppen physiologische Kochsalzlösung.

17 Stunden nach dem Alkoholentzug wurde anhand einer Verhaltenstestbatterie (Punksystem) mit der Bewertung der Entzugssymptome begonnen (Tabelle 8).

Für die statistische Sicherung von Aussagen wurde zum Vergleich von Gruppenmedianwerten der parameterfreie MANN-WHITNEY-Test (U-Test) verwendet.

Tabelle 8

| Punkteverteilung für das Auftreten von Entzugssymptomen nach Majchrowicz | |
|---|---|
| Punkte | Symptome |
| 1 | 1 = STRAUBsches Schwanzphänomen |
| 1 | 2 = Piloerektion |
| 1 | 3 = Bewegungsauffälligkeiten |
| 1 | 4 = Katalepsie, Hinlegen |
| 2 | 5 = Konvulsionen einzelner Muskelgruppen |
| 2 | 6 = Schütteln von Kopf oder Körper |
| 2 | 7 = Zähneklappern |
| 2 | 8 = Tremor |
| 3 | 9 = unnormale Laufbewegungen |
| 3 | 10 = spontane Kovulsionen |
| 3 | 11 = spontane Vokalisation |

Neben den spontan auftretenden Entzugserscheinungen wurden zusätzliche Symptome durch handling oder akustische Reize provoziert und bewertet.

Die erzielten Ergebnisse sind aus der Tabelle 9 zu ersehen. Bel allen drei mit Lys-pyrrolidid behandelten Gruppen konnte im Vergleich zur Placebogruppe eine signifikante Senkung der Gesamtpunktezahl für registrierte Symptome festgestellt werden.

Tabelle 9 Beobachtete Entzugssymptome zu verschiedenen Zeitpunkten nach letzter Alkoholgabe von mit Lys-pyrrolidid behandelten Tieren und Kontrolltieren

| Beobachtung nach Entzug | Gruppe | Tierzahl n | Punkte x | Spannweite R | Vergleich zu Gr. I (U-Test) p | U |
|---|---|---|---|---|---|---|
| 1. (17-21 h) | I NaCl | 11 | 7,0 | 14 | | |
| | II 1xLP(1d) | 10 | 5,0 | 12 | | 37,5 |
| | III 1xLP(1h) | 11 | 5,0 | 1o | | 48,0 |
| | IV 4xLP | 12 | 3,5 | 9 | < 0,05 | 31,5 |
| 2. (15-28 h) | I | 11 | 9,0 | 14 | | |
| | II | 10 | 4,5 | 7 | < 0,02 | 19,0 |
| | III | 11 | 3,0 | 11 | < 0,02 | 20,0 |
| | IV | 12 | 3,5 | 4 | < 0,002 | 12,0 |
| 3. (41 h) | I | 11 | 5,0 | 8 | | |
| | II | 10 | 4,5 | 7 | | 50,5 |
| | III | 11 | 4,0 | 6 | | 38,0 |
| | IV | 12 | 2,5 | 8 | | 35,5 |
| 4. (70 h) | I | 10 | 2,5 | 6 | | |
| | II | 10 | 1,0 | 4 | < 0,05 | 21,5 |
| | III | 11 | 1,0 | 3 | < 0,02 | 19,5 |
| | IV | 12 | 2,0 | 4 | < 0,05 | 25,0 |
| 5. (98 h) | I | 10 | 2,5 | 6 | | |
| | II | 10 | 1,0 | 5 | | 41,5 |
| | III | 11 | 0,0 | 4 | | 31,5 |
| | IV | 12 | 0,0 | 4 | < 0,05 | 27,5 |

Beispiel 13 *Wirksamkeit am allergischen Asthmamodell des narkotisierten Meerschweinchens*

Männlichen Meerschweinchen mit einem Körpergewicht zwischen 350 und 550 g wurde nach Tracheometrie eine Trachealkanüle in die Trachea fest eingebunden und die Tiere in einem Tankrespirator fixiert und nach Relaxation mit 0,2 mg/kg Pancuroniumbromid mit einem rhythmischen Unterdruck von -3 kPa, einer Frequenz von 20/min und einem Inspiration : Expirations-Verhältnis von 1 : 1 beatmet. Die Messung

der Atemparameter Atemfluß V und Atemzugvolumen VT erfolgte pneumotachographisch mit einer Fleisch Düse, einem Differenzdruckwandler und einem Physiointegrator. Die Ovalbuminsensibilisierung erfolgte nach einer Methode von Andersson (Andersson, P., Allergy 35, S. 65-71, 1980) durch eine Injektion (i.p.) von 10 µg Ovalbumin und 100 mg Aluminiumhydroxid. Drei Tage vor dem Versuch erfolgte eine inhalative Kontrolle des Sensibilisierungserfolges durch Vernebelung einer Ovalbuminlösung (1 mg/ml).
Die Applikation von Lys-pyrrolidid erfolgte an 4 aufeinanderfolgenden Tagen einmal täglich mit 4 mg/kg.
Das Lys-pyrrolidid, gelöst in isotonischer NaCl-Lösung (50 µg/ml), wurde oral über eine flexible Magensonde appliziert. Die Auslösung des Bronchospasmus erfolgte durch Ovalbumin (1 mg/kg i. t.).
Der Verlauf von VT nach Ovalbumin-Instillation ist in der Abbildung 2 dargestellt. Bei den mit Lys-pyrrolidid vorbehandelten Tieren wurde im Vergleich mit den Kontrolluntersuchungen ein signifikant verminderter Bronchospasmus nach Allergeninstillation gefunden. Die Mittelwerte der vorbehandelten Gruppe lagen bei allen 6 Versuchspaaren 15-20 % über den Mittelwerten der Kontrollgruppe.
Am allergischen Bronchokonstriktionsmodell an Ovalbuminsensibilisierten Meerschweinchen wurden weitere antiasthmatisch wirkende Substanzen untersucht.
Ein Vergleich mit der erfingungsgemäßen Verbindung ist jedoch kaum möglich, da die Substanzen in einem anderen Applikationsregime (eimalige Gabe, i. v., i. p.) gegeben wurden. Die durch Lys-pyrrolidid erzielte Hemmung des allergisch bedingten Bronchospasmus von 15 bis 20 % entspricht Werten, die z. B. auch durch Lipoxygenaseinhibitoren am allergischen oder durch Arachidonsäure-induzierten Bronchospasmus erreicht werden. Damit ist die Wirkungsstärke dieser Verbindung vergleichbar mit anderen antiasthmatisch wirkenden Substanzen.

Darstellung des mittleren Atemzugvolumens ($V_T$) als prozentualer Anteil des Atemzugvolumens ($V_{Ti}$) vor der Provokation am allergischen Asthmamodell des narkotisierten Meerschweinchens mit Lys-pyrrolidid (LP) und ohne (contol)

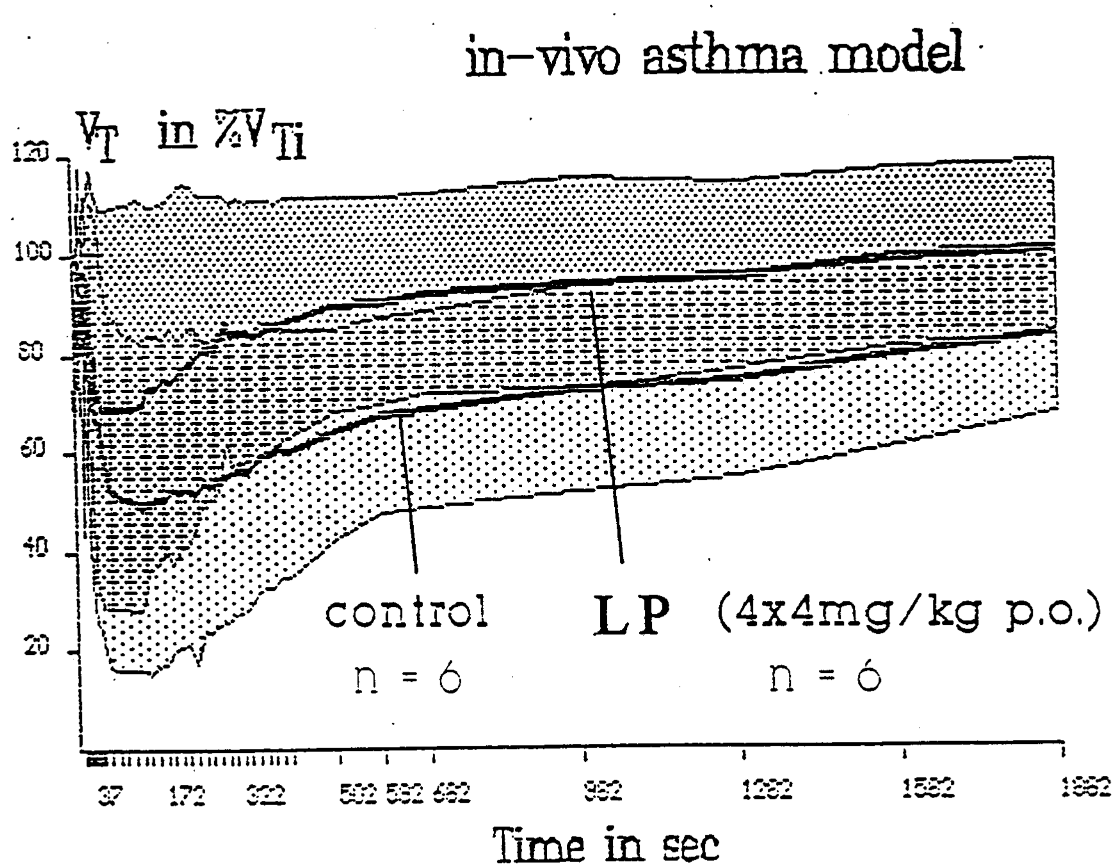


Abbildung 2

Beispiel 14 *Einfluß auf die ACh-induzierten Kontraktionen an Trachea- und Lungenparenchympräparaten von Ovalbumin-sensibilisierten und unbehandelten Tieren*

Die Untersuchungen wurden an spiralig geschnittenen Tracheapräparaten und an Parenchymstreifen des unteren Lungenlappens von Ovalbumin-sensibilisierten (s. Beispiel 13) sowie an unvorbehandelten Tieren durchgeführt. Die Untersuchungen wurden in einem thermostatierten Organbad in physiologischer Lösung bei einer Temperatur von 37 °C durchgeführt. Die Registrierung erfolgte isotonisch über einen reibungsarm gelagerten Waagebalken in Verbindung mit einem Hochfrequenzschwingkreis mit Verstärker zur berührungslosen Wegmessung. Vor Versuchsbeginn wurden die Präparate vorgedehnt und konditioniert. Alle durch ACh oder Ovalbumin induzierten Kontraktionen wurden als prozentualer Anteil, bezogen auf die durch 500 µM ACh induzierten Kontraktionen am gleichen Präparat vor Versuchsbeginn, errechnet.
Bei Vergleichsuntersuchungen (Behandlung/Kontrolle) wurde die prozentuale Hemmung der Kontraktion am Behandlungspräparat, bezogen auf die am zugehörigen Kontrollpräparat, berechnet. Lys-pyrrolidid wurde in

physiologischer Lösung gelöst und direkt in das Organbad gegeben. Bei den Kontrolluntersuchungen wurde das entsprechende Volumen Kochsalzlösung appliziert.
Lys-pyrrolidid wurde in der Konzentration von 1 µM untersucht. Bei dieser Konzentration kommt es zu einer Rechtsverschiebung der ACh-Konzentrations-Wirkungskurve an Trachea- und Lungenparenchympräparaten. Die errechneten $ED_{50}$-Werte sind in Tabelle 10 dargestellt.

Tabelle 10

| $ED_{50}$-Werte für die ACh-induzierten Kontraktionen an Trachea- und Lungenparenchympräparaten mit und ohne Lys-pyrrolidid-Vorbehandlung (1 µM) | | | | |
|---|---|---|---|---|
| Präparat | $ED_{50}$ | | | |
| | Kontrolle | n | Lys-pyrroldid | n |
| Lunge sensibil. | 8 µM | 4 | 650 µM | 4 |
| Trachea sensibil. | 0,1 µM | 4 | 1 µM | 4 |
| Lunge o. Vorbeh. | 7 µM | 4 | 7,2 µM | 4 |
| Trachea o. Vorbeh. | 0,3 µM | 4 | 0,3 µM | 4 |

Die Ergebnisse in Tabelle 10 zeigen, daß der erfindungsgemäße Wirkstoff nur eine hemmende Wirkung an Präparaten von sensibilisierten Tieren zeigt. An Präparaten von unbehandelten Tieren war die Substanz in der untersuchten Konzentration wirkungslos.

Beispiel 15 *Einfluß auf die durch Ovalbumin induzierten Kontraktionen an Trachea- und Lungenparenchympräparaten von Ovalbumin-sensibilisierten Tieren*

Die Untersuchungen wurden analog dem Beispiel 14 an Präparaten von Ovalbumin-sensibilisierten Tieren durchgeführt. Die Kontraktionen wurden durch 0,1 mM Ovalbumin hervorgerufen. Lys-pyrrolidid wurde in einer Konzentration von 1 µM untersucht.
Die Ergebnisse sind in Tabelle 11 dargestellt.

Tabelle 11

| Einfluß von Lys-pyrrolidid (1 µM) auf die Ovalbumininduzierten Kontraktionen an Trachea- und Lungenparenchympräparaten von sensibilisierten Meerschweinchen (Mittelwerte ±SEM) | | | | |
|---|---|---|---|---|
| Präparat | Kontrolle | n | Lys-pyrrolidid | n |
| | (% der durch 500 µM ACh ind. Kontraktion) | | | |
| Lunge | 184,1 ± 11,4 | 4 | 141,5 ± 15,0* | 4 |
| Trachea | 63,4 ± 10,1 | 4 | 48,3 ± 12,2** | 4 |

* p < o,o1
** p < 0,05

Die Ergebnisse zeigen, daß Lys-pyrrolidid in der untersuchten Konzentration die durch Ovalbumin induzierten Kontraktionen an Lungen- und Tracheapräparaten hemmt.

Beispiel 16 *Einfluß auf die elektrisch evozierte ACh-Freisetzung aus Tracheaschnitten von Ovalbumin-sensibilisierten Tieren*

Die Untersuchungen wurden an Tracheaschnitten (0,3 mm dick) von Ovalbumin-sensibilisierten Tieren (s. Beispiel 13) durchgeführt. Die Schnitte wurden 30 min mit Methyl-3H-cholin-chlorid (80 Ci/mol) in einer Konzentration von 0,1 µM vorbehandelt. Anschließend wurden die Schnitte in einer Superfusionskammer kontinuierlich mit physiologischer Lösung (0,5 ml/min) superfundiert. Das Superfusat wurde in 1-min-Fraktionen gesammelt. Die Bestimmung der freigesetzten Transmittermenge erfolgte durch Bestimmung der Radioaktivität im Superfusat mittels Flüssigkeitsszintillationsspektrometrie. Die Stimulation der Schnitte erfolgte mit Rechteckimpulsen über zwei parallel angeordnete Platinelektroden, die mit einem Stimulator

verbunden waren. Folgende Stimulationsparameter wurden eingesetzt:
Frequenz = 30 Hz; Amplitude = 30 V; Stimulationszeit = 60 s; Impulsbreite = 1 ms.
Lys-pyrrolidid wurde in entsprechender Konzentration direkt in die Superfusionslösung gegeben.
In allen Untersuchungen wurde die evozierte 3H-ACh-Freisetzung als prozentualer Anteil der gesamten gespeicherten Radioaktivität zum Zeitpunkt des Einsetzens des Stimulus berechnet und als Freisetzungsrate dargestellt.

Tabelle 12

| Einfluß von Lys-pyrrolidid auf die elektrisch evozierte ACh-Freisetzung aus Tracheaschnitten von Ovalbuminsensibilisierten Meerschweinchen, dargestellt an Hand der prozentualen Freisetzungsrate (Mittelwerte ±SEM) | | | |
|---|---|---|---|
| Vorbehandlung | Konzentration ( µM) | Freisetzungsrate (%) | n |
| Kontrolle | | 1,1 ± 0,2 | 9 |
| + Lys-pyrrolidid | 0,1 | 0,4 ± 0,1* | 9 |
| + Lys-pyrrolidid | 1,0 | 0,6 ± 0,1* | 7 |
| + Lys-pyrrolidid | 10,0 | 0,7 ± 0,1** | 9 |

* $p < 0{,}01$
** $p < 0{,}05$

Die Ergebnisse belegen, daß die erfindungsgemäße Verbindung in dem untersuchten Konzentrationsbereich die elektrisch evozierte ACh-Freisetzung aus Tracheaschnitten von Ovalbumin-sensibilisierten Meerschweinchen hemmt.

## Patentansprüche

**1.** Chemische Verbindung der Formel I

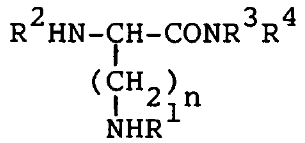

I,

worin n gleich 2 bis 4 ist, $R^1$ und $R^2$ unabhängig voneinander Wasserstoff oder Acylreste, $R^3$ und $R^4$ unabhängig voneinander Alkyl oder Aralkyl bedeuten oder $R^3$ und $R^4$ gemeinsam mit dem Stickstoffatom, an dem sie gebunden sind, einen ggf. substituierten heterocyclischen Ring bilden oder deren pharmazeutisch verträgliche Salze.

**2.** Chemische Verbindung nach Anspruch 1, dadurch gekennzeichnet, daß $R^3$ und $R^4$ gemeinsam mit dem Stickstoffatom, an dem sie gebunden sind, einen heterocyclischen Ring, insbesondere einen fünf- oder sechsgliedrigen heterocyclischen Ring, bilden, der ggf. durch eine Carboxylgruppe substituiert ist und/oder ggf. weitere Heteroatome, insbesondere Sauerstoff oder Schwefel enthält.

**3.** Chemische Verbindung nach den Ansprüchen 1 oder 2, dadurch gekennzeichnet, daß $R^3$ und $R^4$ gemeinsam mit dem Stickstoffatom, an dem sie gebunden sind, einen fünfgliedrigen heterocyclischen Ring bilden, der ggf. durch eine Carboxylgruppe substituiert ist und/oder ggf. als weiteres Heteroatom Schwefel enthält.

**4.** Chemische Verbindung nach einem der Ansprüche 1-3, dadurch gekennzeichnet, daß $R^3$ und $R^4$ gemeinsam mit dem Stickstoffatom, an dem sie gebunden sind, einen sechsgliedrigen heterocyclischen Ring bilden, der ggf. als weiteres Heteroatom Sauerstoff enthält.

**5.** Chemische Verbindung nach einem der Ansprüche 1-4, dadurch gekennzeichnet, daß $R^1$ und/oder $R^2$ einen Acetyl-, Propionyl- oder tert. Pentanoylrest, vorzugsweise $R^1$ einen Acetylrest und $R^2$ Wasserstoff, bedeuten.

**6.** Chemische Verbindung nach einem der Ansprüche 1-5, dadurch gekennzeichnet, daß n gleich 3 oder 4, vorzugsweise jedoch 4 ist.

**7.** Chemische Verbindung nach einem der Ansprüche 1-6, dadurch gekennzeichnet, daß $NR^3R^4$ Prolin, Pyrrolidin oder Morpholin, vorzugsweise jedoch Pyrrolidin bedeutet.

**8.** Chemische Verbindung nach einem der Ansprüche 1-7. dadurch gekennzeichnet, daß n gleich 4, $R^1$ Wasserstoff oder Acetyl, $R^2$ Wasserstoff und $-NR^3R^4$ Pyrrolidin bedeuten.

**9.** Chemische Verbindung nach einem der Ansprüche 1-8, dadurch gekennzeichnet, daß $R^3$ und $R^4$ Alkyl- oder Aralkylreste mit 1 bis 10 Kohlenstoffatomen bedeuten.

**10.** Chemische Verbindung nach einem der Ansprüche 1-9, in Form ihrer Salze von Mineralsäuren, insbesondere als Hydrochloride oder als Salze von organischen Säuren, insbesondere von Dicarbonsäuren.

**11.** Pharmazeutische Zusammensetzungen, dadurch gekennzeichnet, daß sie als Wirkstoff eine oder mehrere der Verbindungen der Formel I, wie in einem der Ansprüche 1 bis 10 definiert, oder ein oder mehrere pharmazeutisch verträgliche Salze davon enthalten.

**12.** Verwendung einer chemischen Verbindung der Formel I, wie in einem der Ansprüche 1 bis 10 definiert, oder eines pharmazeutisch verträglichen Salzes davon zur Herstellung eines Arzneimittels zur Anwendung als Antistreßmittel bei der parenteralen und oralen Prophylaxe und Therapie von durch chronischen Streß bedingten funktionellen und vegetativen Störungen und zur Verbesserung der Adaptionsfähigkeit gegenüber chronischen Stressor-Einflüssen.

**13.** Verwendung einer chemischen Verbindung der Formel I oder eines pharmazeutisch verträglichen Salzes davon nach Anspruch 12, dadurch gekennzeichnet, daß als Stressoren Dauerbelastung durch Immobilisation, Konfliktsituationen, traumatische Faktoren, Alkohol und Suchtmittel bzw. deren Entzug wirken.

**14.** Verwendung einer chemischen Verbindung der Formel 1, wie in einem der Ansprüche 1 bis 10 definiert, oder eines pharmazeutisch verträglichen Salzes davon zur Herstellung eines Arzneimittels zur Behandlung von Alkoholismus und dessen Entzugserscheinungen.

**15.** Verwendung einer chemischen Verbindung der Formel I nach einem der Ansprüche 1 bis 10 oder eines pharmazeutisch verträglichen Salzes davon zur Herstellung eines Arzneimittels zur Antisuchtbehandlung.

**16.** Verwendung einer chemischen Verbindung der Formel I nach einem der Ansprüche 1 bis 10 oder eines pharmazeutisch verträglichen Salzes davon zur Herstellung eines Arzneimittels zur Behandlung von Störungen der Lern- und Gedächtnisleistungen.

**17.** Verwendung einer chemischen Verbindung der Formel I nach einem der Ansprüche 1 bis 10 oder eines pharmazeutisch verträglichen Salzes davon zur Herstellung eines Antiasthmatikums für die Therapie von allergisch bedingten Störungen der Atemwege.

**Patentansprüche für folgende Vertragsstaaten : ES, GR**

**1.** Verfahren zur Herstellung einer pharmazeutischen Zusammensetzung, dadurch gekennzeichnet, daß eine chemische Verbindung der Formel I

$$\begin{array}{l} R^2HN\text{-}CH\text{-}CONR^3R^4 \\ \quad\quad\;\; | \\ \quad\;\; (CH_2)n \\ \quad\quad\;\; | \\ \quad\;\; NHR^1 \end{array} \qquad I,$$

worin n gleich 2 bis 4 ist, $R^1$ und $R^2$ unabhängig voneinander Wasserstoff oder Acylreste, $R^3$ und $R^4$ unabhängig voneinander Alkyl oder Aralkyl bedeuten oder $R^3$ und $R^4$ gemeinsam mit dem Stickstoffatom, an dem sie gebunden sind, einen ggf. substituierten heterocyclischen Ring bilden, oder mehrere chemische Verbindungen der Formel I oder deren pharmazeutisch verträgliches Salz oder deren pharmazeutisch verträglichen Salze mit Träger und/oder Zusatzstoffen gemischt oder in geeigneten Flüssigkeiten gelöst werden.

**2.** Verfahren zur Herstellung einer pharmazeutischen Zusammensetzung nach Anspruch 1, dadurch gekennzeichnet, daß $R^3$ und $R^4$ gemeinsam mit dem Stickstoffatom, an dem sie gebunden sind, einen heterocyclischen Ring, insbesondere einen fünf- oder sechsgliedrigen heterocyclischen Ring, bilden, der ggf. durch eine Carboxylgruppe substituiert ist und /oder ggf. weitere Heteroatome, insbesondere Sauerstoff oder Schwefel enthält.

**3.** Verfahren zur Herstellung einer pharmazeutischen Zusammensetzung nach einem der Ansprüche 1 bis 2, dadurch gekennzeichnet, daß $R^3$ und $R^4$ gemeinsam mit dem Stickstoffatom, an dem sie gebunden sind, einen fünfgliedrigen heterocyclischen Ring bilden, der ggf. durch eine Carboxylgruppe substituiert ist und/oder ggf. als weiteres Heteroatom Schwefel enthält.

**4.** Verfahren zur Herstellung einer pharmazeutischen Zusammensetzung nach einem der Ansprüche 1 bis

3, dadurch gekennzeichnet, daß $R^3$ und $R^4$ gemeinsam mit dem Stickstoffatom, an dem sie gebunden sind, einen sechsgliedrigen heterocyclischen Ring bilden, der ggf. als weiteres Heteroatom Sauerstoff enthält

**5.** Verfahren zur Herstellung einer pharmazeutischen Zusammensetzung nach einem der Ansprüche 1 bis 4, dadurch gekennzeichnet, daß $R^1$ und/oder $R^2$ einen Acetyl-, Propionyl- oder tert. Pentanoylrest, vorzugsweise $R^1$ einen Acetylrest und $R^2$ Wasserstoff, bedeuten.

**6.** Verfahren zur Herstellung einer pharmazeutischen Zusammensetzung nach einem der Ansprüche 1 bis 5, dadurch gekennzeichnet, daß n gleich 3 oder 4, vorzugsweise jedoch 4 ist.

**7.** Verfahren zur Herstellung einer pharmazeutischen Zusammensetzung nach einem der Ansprüche 1 bis 6, dadurch gekennzeichnet, daß $NR^3R^4$ Prolin, Pyrrolidin oder Morpholin, vorzugsweise jedoch Pyrrolidin bedeutet.

**8.** Verfahren zur Herstellung einer pharmazeutischen Zusammensetzung nach einem der Ansprüche 1 bis 7, dadurch gekennzeichnet, daß n gleich 4, $R^1$ Wasserstoff oder Acetyl, $R^2$ Wasserstoff und $-NR^3R^4$ Pyrrolidin bedeuten.

**9.** Verfahren zur Herstellung einer pharmazeutischen Zusammensetzung nach einem der Ansprüche 1 bis 8, dadurch gekennzeichnet, daß $R^3$ und $R^4$ Alkyl- oder Aralkylreste mit 1 bis 10 Kohlenstoffatomen bedeuten.

**10.** Verfahren zur Herstellung einer pharmazeutischen Zusammensetzung nach einem der Ansprüche 1 bis 9, dadurch gekennzeichnet, daß die chemischen Verbindungen der Formel 1 als Salze von Mineralsäuren, insbesondere als Hydrochloride oder als Salze von organischen Säuren, insbesondere von Dicarbonsäuren, eingesetzt werden.

**11.** Verfahren zur Herstellung einer pharmazeutischen Zusammensetzung nach einem der Ansprüche 1 bis 10 zur parenteralen und oralen Prophylaxe und Therapie von durch chronischen Streß bedingten funktionellen und vegetativen Störungen und zur Verbesserung der Adaptionsfähigkeit gegenüber chronischen Stressor-Einflüssen.

**12.** Verfahren zur Herstellung einer pharmazeutischen Zusammensetzung nach Anspruch 11, dadurch gekennzeichnet, daß als Stressoren Dauerbelastung durch Immobilisation, Konfliktsituationen, traumatische Faktoren, Alkohol und Suchtmittel bzw. deren Entzug wirken.

**13.** Verfahren zur Herstellung einer pharmazeutischen Zusammensetzung nach einem der Ansprüche 1 bis 10 zur Behandlung des Alkoholismus und dessen Entzugserscheinungen.

**14.** Verfahren zur Herstellung einer pharmazeutischen Zusammensetzung nach einem der Ansprüche 1 bis 10 als Antisuchtmittel.

**15.** Verfahren zur Herstellung einer pharmazeutischen Zusammensetzung nach einem der Ansprüche 1 bis 10 zur Behandlung von Störungen der Lern- und Gedächtnisleistungen.

**16.** Verfahren zur Herstellung einer pharmazeutischen Zusammensetzung nach einem der Ansprüche 1 bis 10 als Antiasthmatikum für die Therapie von allergisch bedingten Störungen der Atemwege.

**17.** Verwendung einer chemischen Verbindung der Formel I nach einem der Ansprüche 1 bis 10 oder eines pharmazeutisch verträglichen Salzes davon zur Herstellung eines Arzneimittels zur parenteralen und oralen Prophylaxe und Therapie von durch chronischen Streß bedingten funktionellen und vegetativen Störungen und zur Verbesserung der Adaptionsfähigkeit gegenüber chronischen Stressor-Einflüssen.

**18.** Verwendung einer chemischen Verbindung nach Anspruch 17, dadurch gekennzeichnet, daß als Stressoren Dauerbelastung durch Immobilisation, Konfliktsituationen, traumatische Faktoren, Alkohol und Suchtmittel bzw. deren Entzug wirken.

19. Verwendung einer chemischen Verbindung der Formel I nach einem der Ansprüche 1 bis 10 oder eines pharmazeutisch verträglichen Salzes davon zur Herstellung eines Arzneimittels zur Behandlung von Alkoholismus und dessen Entzugserscheinungen.

20. Verwendung einer chemischen Verbindung der Formel I nach einem der Ansprüche 1 bis 10 oder eines pharmazeutisch verträglichen Salzes davon zur Herstellung eines Arzneimittels zur Antisuchtbehandlung.

21. Verwendung einer chemischen Verbindung der Formel I nach einem der Ansprüche 1 bis 10 oder eines pharmazeutisch verträglichen Salzes davon zur Herstellung eines Arzneimittels zur Behandlung von Störungen der Lern- und Gedächtnisleistungen.

22. Verwendung einer chemischen Verbindung der Formel I nach einem der Ansprüche 1 bis 10 oder eines pharmazeutisch verträglichen Salzes davon zur Herstellung eines Antiasthmatikums bei der Therapie von allergisch bedingten Störungen der Atemwege.

Fig. 1

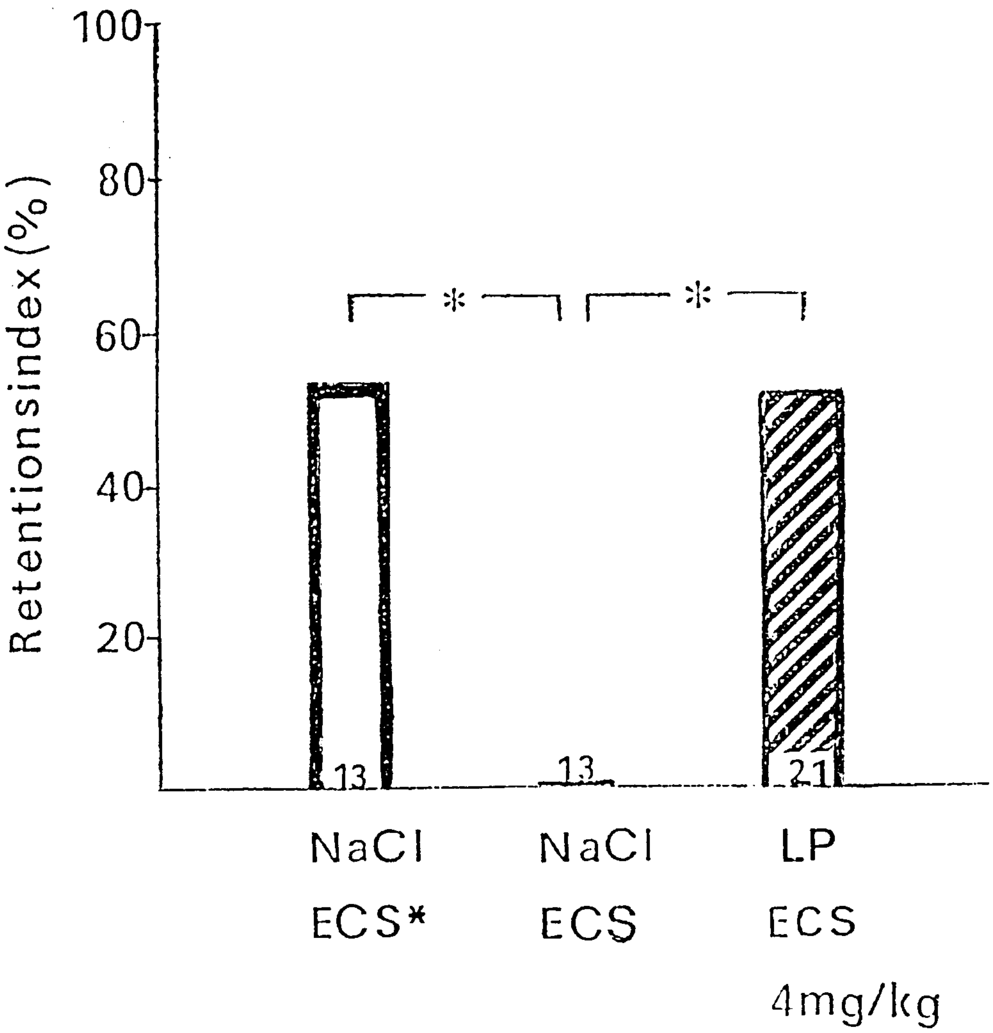
100
80
60
40
20
Retentionsindex (%)
*
*
13
13
21
NaCl
ECS*
NaCl
ECS
LP
ECS
4mg/kg

Fig. 2

Darstellung des mittleren Atemzugvolumens ($V_T$) als prozentualer Anteil des Atemzugvolumens ($V_{Ti}$) vor der Provokation am allergischen Asthmamodell des narkotisierten Meerschweinchens mit Lys-pyrrolidid (LP) und ohne (contol)